(19) Europäisches Patentamt
European Patent Office
Office européen des brevets

(11) EP 2 861 301 B1

(12) **FASCICULE DE BREVET EUROPEEN**

(45) Date de publication et mention de la délivrance du brevet:
**20.02.2019 Bulletin 2019/08**

(21) Numéro de dépôt: **13733396.9**

(22) Date de dépôt: **13.06.2013**

(51) Int Cl.:
***A61P 19/02*** *(2006.01)* ***A61K 8/60*** *(2006.01)*
***A61K 8/73*** *(2006.01)* ***A61Q 19/08*** *(2006.01)*
***A61K 31/7016*** *(2006.01)* ***A61K 31/728*** *(2006.01)*
***A61P 27/04*** *(2006.01)* ***A61K 47/36*** *(2006.01)*
***A61K 45/06*** *(2006.01)* ***A61K 31/167*** *(2006.01)*

(86) Numéro de dépôt international:
**PCT/FR2013/051380**

(87) Numéro de publication internationale:
**WO 2013/186493 (19.12.2013 Gazette 2013/51)**

(54) **COMPOSITION, EN MILIEUX AQUEUX, COMPRENANT AU MOINS UN ACIDE HYALURONIQUE ET AU MOINS UN SEL HYDROSOLUBLE DE SUCROSE OCTASULFATE**

ZUSAMMENSETZUNG IN EINEM WÄSSRIGEN MEDIUM MIT MINDESTENS EINER HYALURONSÄURE UND MINDESTENS EINEM WASSERLÖSLICHEN SACCHAROSE-OCTASULPHAT-SALZ

COMPOSITION, IN AN AQUEOUS MEDIUM, INCLUDING AT LEAST ONE HYALURONIC ACID AND AT LEAST ONE SUCROSE OCTASULPHATE WATER-SOLUBLE SALT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.06.2012 FR 1255543**
**13.06.2012 US 201261659140 P**

(43) Date de publication de la demande:
**22.04.2015 Bulletin 2015/17**

(73) Titulaire: **Laboratoires Vivacy**
**74160 Archamps (FR)**

(72) Inventeurs:
- **PIRON, Estelle**
**F-73490 La Ravoire (FR)**
- **BON BETEMPS, Jérémie**
**F-73410 Albens (FR)**

(74) Mandataire: **Tripoz, Inès**
**Cabinet Tripoz**
**Le Pôle Sud**
**22 rue Seguin**
**69002 Lyon (FR)**

(56) Documents cités:
**EP-A1- 0 640 346** **EP-A2- 2 070 518**
**WO-A2-03/000191**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 861 301 B1

## Description

L'acide hyaluronique est utilisé depuis plus de quinze ans dans le domaine de l'esthétique, où il a prouvé son innocuité et son efficacité. A ce jour, sur le marché des gels de comblement à visée esthétique ou « fillers », les gels à base d'acide hyaluronique réticulé d'origine biofermentaire sont les produits les plus utilisés. En effet, dans le domaine de l'esthétique, les implants non résorbables sont de moins en moins utilisés et les gels à base d'autres polysaccharides ont été abandonnés soit à cause d'effets secondaires, soit à cause de leur origine animale entraînant un risque de contamination virale.

L'utilisation de l'acide hyaluronique d'origine biofermentaire dans les domaines tels que le comblement de rides, la viscosupplémentation, le traitement ophtalmique ou encore le traitement de l'incontinence urinaire est d'autant plus reconnue et appréciée que de par sa présence naturelle dans le corps humain, et plus particulièrement dans le derme, le liquide synovial et la cornée, les risques dus aux effets secondaires sont minimisés.

Mais du fait que l'acide hyaluronique est présent naturellement dans le corps, il existe des enzymes endogènes susceptibles de le dégrader, comme par exemple les hyaluronidases. Cette dégradation est illustrée dans Iocono et al., J. Ped. Surg., Vol. 33, No. 4, 1988, 564-567, qui traite de la cicatrisation des plaies. Les hyaluronidases sont des enzymes qui dégradent l'acide hyaluronique en catalysant son hydrolyse en oligosaccharides d'acide hyaluronique. Cette dégradation a pour effet une diminution de la viscosité de l'acide hyaluronique. Cette diminution de viscosité entraîne irrémédiablement une diminution dans le temps des effets recherchés dans les domaines du comblement de rides ou de la viscosupplémentation, d'où un rapprochement dans le temps des injections pour en restaurer les effets, susceptibles d'entraîner une gêne ou des douleurs pour le patient.

Une première solution pour résoudre ce problème pourrait être de substituer d'autres polysaccharides, moins sensibles aux hyaluronidases et naturellement présents dans le corps humain, à l'acide hyaluronique.

Parmi les polysaccharides constitutifs de la Matrice ExtraCellulaire (MEC), la Chondroïtine sulfate, le Dermatane sulfate et la Kératane sulfate pourraient être intéressants.

Ils sont en effet responsables en partie avec l'acide hyaluronique de l'hydratation de la peau, de sa souplesse, et leur présence naturelle dans le derme permet de prévoir une excellente tolérance et efficacité pour un produit de comblement de rides, de réhydratation ou de viscosupplémentation.

Malheureusement, la chondroïtine sulfate est d'origine animale et aucune tentative de synthèse de la chondroïtine sulfate par voie biofermentaire n'a, à ce jour, permis de la produire en des quantités compatibles avec une exploitation industrielle. Il en est de même pour le dermatane sulfate et la kératane sulfate, qui ne sont disponibles industriellement que comme produits d'origine animale.

Une autre solution visant à diminuer la dégradation de l'acide hyaluronique par les hyaluronidases est de proposer des compositions comprenant en combinaison avec l'acide hyaluronique, un composé ayant les propriétés inhibitrices des hyaluronidases.

Les groupements sulfates sont connus dans la littérature pour leurs propriétés anti-oxydante et anti-radicalaire, pour leur capacité d'hydratation importante et également pour leur action anti-hyaluronidase.

S'agissant de leurs propriétés anti-oxydante et anti-radicalaire, ils protègent les glycosaminoglycanes (GAGs) de la dépolymérisation par les espèces réactives de l'oxygène comme décrit dans Moseley R et al., Biochim Biophys Acta., (1995), 1244(2-3), 245-52.

S'agissant de leur capacité d'hydratation, il a été démontré par exemple dans R. Servaty et al., International Journal of Biological Macromolecules, (2001), 28, 121-127, une plus grande capacité d'absorption des molécules d'eau par la chondroïtine sulfate par rapport à l'acide hyaluronique résultant de la présence du groupement sulfate porté par la chondroïtine sulfate.

S'agissant de leur action anti-hyaluronidase, les polysaccharides sulfatés et plus particulièrement les glycosaminoglycanes entièrement O-sulfatés sont connus pour présenter une activité anti-hyaluronidase importante, par exemple de T. Toida et al., Archives of Biochemistry and Biophysics, 1999, 370(2), 176-182. Les glycosaminoglycanes entièrement O-sulfatés décrits dans cette publication sont la chondroïtine sulfate, le dermatane sulfate, l'héparine sulfate, et l'acide hyaluronique sulfaté. Les résultats obtenus montrent que tous les glycosaminoglycanes entièrement O-sulfatés inhibent les hyaluronidases de manière dose-dépendante et que l'acide hyaluronique entièrement O-sulfaté est le meilleur inhibiteur des hyaluronidases parmi les glycosaminoglycanes entièrement O-sulfatés testés. L'inconvénient de l'acide hyaluronique entièrement O-sulfaté est qu'il ne peut être réticulé.

Or, dans le domaine de l'esthétique, plus de 80 % des compositions sont à base d'acide hyaluronique réticulé, il n'est donc pas possible de développer un produit de comblement à base d'acide hyaluronique non réticulé.

On pourrait envisager l'incorporation d'une petite quantité de polysaccharide sulfaté d'origine animale dans une composition à base d'acide hyaluronique, par exemple la demande de brevet WO 03/041724 au nom de Hermida, décrit des compositions injectables stériles à base d'un mélange de hyaluronate de sodium et de chondroïtine sulfate à visée intraarticulaire.

De même, dans la demande de brevet WO 2004/034980 au nom de Marcum, des compositions injectables à

base d'acide hyaluronique, de chondroïtine sulfate et de N-acétyl-glucosamine pour la régénération du cartilage par injection intraarticulaire sont décrites.

L'inconvénient de telles compositions demeure l'origine animale de la chondroïtine sulfate.

S'agissant des polysaccharides sulfatés d'origine synthétique, des tests d'inhibition des hyaluronidases dans des compositions d'acide hyaluronique par le dextrane sulfate ont également été décrits, par exemple par K. Zimmermann, J. Cancer Res. Clin. Oncol., 1983, 105 pp 189-190. L'hydrolyse de l'acide hyaluronique selon la méthode de Nelson a été mesurée pour des compositions comprenant de l'acide hyaluronique, des hyaluronidases ainsi que du dextrane sulfate de poids moléculaire de 17,7 kDa à différentes concentrations. Le dextrane sulfate est décrit comme un puissant inhibiteur des hyaluronidases, et si les doses actives pour un dextrane sulfate de poids moléculaire de 17,7 kDa sont éloignées des doses toxiques, la toxicité augmente avec le poids moléculaire, et seules quelques molécules de haut poids moléculaire augmentent considérablement la toxicité, comme cela a été démontré par Walton, Br. J. Pharmacol., 1954, 9 :1-14. De plus le dextrane sulfate est décrit comme étant un anticoagulant utilisable *in vivo* et *in vitro* comme cela est décrit par exemple dans US 2715091 et ces propriétés anticoagulantes ne sont a priori pas compatibles avec les utilisations en tant qu'ingrédient de compositions qui sont implantables.

Parmi les polysaccharides d'origine végétale sulfatés, on connait les carraghénanes, mais ceux-ci sont connus pour provoquer des réactions inflammatoires, à tel point que des modèles pour tester les anti-inflammatoires sont basés sur ses propriétés.

On connait également de WO 03/000191 au nom de Depuy, des compositions pour le traitement des articulations arthritiques comprenant de l'acide hyaluronique et un inhibiteur de hyaluronidase, comme par exemple un polysaccharide sulfaté comme le dextrane sulfate, le xylose sulfate ou l'héparane sulfate, dont une partie des composants est encapsulée dans des liposomes pour en assurer la diffusion contrôlée, mais l'utilisation des liposomes dans des compositions destinées notamment à être réticulées semble difficile à envisager.

Ainsi, la plupart des polysaccharides qui pourraient être utilisés au titre d'additifs à l'acide hyaluronique pour diminuer l'action des hyaluronidases sont soit d'origine animale, soit présentent une toxicité potentielle, ces deux conditions étant non compatibles avec les utilisations envisagées.

D'un point de vue théorique, l'incorporation de sulfates dans une solution sous forme d'ions sulfates pourrait être envisagée ; cependant leur diffusion au sein du tissu injecté serait beaucoup trop rapide voir immédiate, donc inefficace pour les propriétés anti-hyaluronidases des compositions dans le domaine du comblement de rides ou de la viscosupplémentation. Il est donc préférable de choisir des composés fonctionnalisés par des groupements sulfates.

Une autre solution potentielle est l'utilisation de composés sulfatés appartenant aux sous-familles des monosaccharides sulfatés et des oligosaccharides sulfatés.

Cette sous-famille de mono ou oligosaccharides sulfatés (formés de 1 à 10 oses) est intéressante, car ils peuvent être facilement disponibles par synthèse, et leur structure glucidique se rapprochant néanmoins des glycosaminoglycanes constitutifs de la Matrice ExtraCellulaire, ils sont très bien métabolisés tout en générant des produits de dégradation non toxiques.

Par exemple, des galacto-oligosaccharides actifs vis-à-vis des hyaluronidases ont été décrits dans les travaux publiés par S. Salmen et al., Planta Medica, 71 n°8, 2005, 727-732, notamment le verbascose, le plantéose et la néomycine, qui présentent des activités anti-hyaluronidases 100 à 500 fois plus élevées que celle de l'apigénine, qui est un inhibiteur de hyaluronidase connu.

Les propriétés anti-hyaluronidases d'oligosaccharides entièrement O-sulfatés provenant de l'acide hyaluronique sont également décrits dans l'art antérieur comme A. Suzuki et al., Glycobiology, 2001, 11(1), pp.57-64. Une corrélation entre l'inhibition des hyaluronidases par des oligosaccharides O-sulfatés provenant de l'acide hyaluronique, leur degré de fonctionnalisation par des groupements sulfates et leur taille est mise en évidence. Il est plus particulièrement mentionné que l'activité inhibitrice de ces oligosaccharides O-sulfatés est liée à leur taille.

Il existe également des monosaccharides sulfatés tels que les glucosamines sulfatées, comme par exemple la glucosamine-3-Sulfate décrite comme inhibiteur de la dégradation du cartilage par J.I. Fenton et al., Osteoarthritis and Cartilage, Volume 8, Issue 6, 2000, 444-451.

Parmi les oligosaccharides, on trouve également le sucrose, formé par la condensation de 2 oses, un glucose et un fructose.

Ce sucrose sous forme sulfatée, comme d'autres oligosaccharides sulfatés, est décrit comme étant utilisé comme agent de promotion de la cicatrisation de plaies en application topique, en solution ou dans des matrices collagéniques ou à base d'alcool polyvinylique dans le brevet EP 0 230 023 au nom de Marion Laboratories.

Dans WO 89/05646 et EP 0640346 au nom de Bar-Shalom, des compositions comprenant des sels de sucrose octasulfate et plus particulièrement des exemples avec le sel d'aluminium de sucrose octasulfate qui est un sel insoluble destinées à des applications topiques ou des injections dans les tissus y compris les articulations sont décrites. Les sels de sucrose octasulfate sont utilisés plus particulièrement pour leurs propriétés cicatrisantes. Dans le cadre de l'application en cicatrisation, l'acide hyaluronique peut également être utilisé en tant qu'actif favorisant la cicatrisation.

On connait de WO 98/22114 au nom de Dumex-Alpharma, des compositions comprenant un complexe formé

entre un polysaccharide tel que le chitosane ou l'acide hyaluronique et un oligosaccharide sulfaté tel que le sucrose octasulfate ou l'un de ses sels. Ces compositions, là encore, sont destinées à favoriser la cicatrisation des plaies des tissus comprenant du collagène tels que la peau, les os et les muqueuses. La formation du complexe est caractérisée par différentes techniques comme par exemple les spectroscopies RMN et infrarouge, la diffraction des rayons X, la thermographie et des essais de solubilité dans des conditions définies.

La fabrication de compositions destinées à être implantées ou injectées implique que celles-ci soient lors de leur processus de fabrication stérilisées pour respecter les exigences sanitaires minimales pour de telles compositions. Le procédé de stérilisation le plus répandu dans les processus de fabrication de telles compositions est la stérilisation à la vapeur ou autoclavage et un tel procédé peut dégrader les produits constituant les compositions. Il est donc indispensable que les propriétés rhéologiques, pH métriques et de biocompatibilité soient conservées et préservées pendant l'autoclavage. Il en est de même s'agissant des exigences d'injectabilité à travers de fines aiguilles, qui est leur mode principal de mise en oeuvre.

Il a été mis en évidence de façon tout à fait surprenante et inattendue par des tests, que les gels d'acide hyaluronique formulés avec les sels hydrosolubles de sucrose octasulfate (SOS) parmi tous les candidats possible sont les seuls qui permettent d'obtenir des compositions stables durant la stérilisation à la vapeur, autrement appelée autoclavage, et que ceux-ci présentent en outre des rémanences accrues en raison de leur résistance à la dégradation par les hyaluronidases, par rapport aux gels comprenant de l'acide hyaluronique sans sel hydrosoluble de sucrose octasulfate.

Le sel hydrosoluble de sucrose octasulfate possède 8 groupements sulfates par molécule de sucrose, ainsi, dans le gel formulé avec du sel hydrosoluble de sucrose octasulfate la quantité de sulfates ajoutée par mg de SOS est importante. La demanderesse a également mis en évidence de manière tout aussi surprenante, que l'addition de sel hydrosoluble de sucrose octasulfate dans des gels à base d'acide hyaluronique n'entraîne pas de chute de viscosité ou d'élasticité du gel durant la stérilisation.

Dans la présente invention, la demanderesse se propose de résoudre le problème de la dégradation par les hyaluronidases de compositions comprenant de l'acide hyaluronique par l'addition d'un sel hydrosoluble de sucrose octasulfate, tout en conservant l'aptitude de ces compositions à être utilisées dans le domaine du comblement de rides, de la viscosupplémentation, des formulations cosmétiques ou pharmaceutiques du fait de la stabilité de leurs propriétés rhéologiques durant la phase de stérilisation à la vapeur au cours du processus de fabrication.

La présente invention permet de résoudre l'ensemble des problèmes cités ci-dessus et permet en outre d'obtenir des compositions selon l'invention conservant leurs propriétés rhéologiques pendant l'autoclavage et ayant une rémanence supérieure (temps de demi-vie supérieur) par rapport aux compositions de l'art antérieur.

La présente invention concerne une composition stérilisée comprenant au moins un acide hyaluronique, réticulé ou non réticulé, ou l'un de ses sels, et au moins un sel hydrosoluble de sucrose octasulfate, les procédés de fabrication de ladite composition ainsi que l'utilisation de ladite composition pour la formulation d'une composition de viscosupplémentation ou pour la formulation d'une composition pour le comblement de rides, pour la formulation d'une composition cosmétique ou pour la formulation d'une composition pharmaceutique.

La composition selon la présente invention possède des propriétés de résistance aux hyaluronidases et est caractérisée par des propriétés rhéologiques conservées après la stérilisation à la vapeur et au moins similaires à celles d'une composition comprenant seulement au moins un acide hyaluronique, réticulé ou non réticulé, ou l'un de ses sels.

La présente invention concerne également une formulation cosmétique comprenant la composition selon l'invention et au moins un excipient cosmétiquement acceptable.

De façon surprenante, la présente invention permet d'obtenir des compositions résistantes aux hyaluronidases répondant aux exigences de stabilité à l'autoclavage des compositions destinées aux domaines précédemment cités.

La présente invention concerne une composition, en milieu aqueux, comprenant au moins un acide hyaluronique et au moins un sel hydrosoluble de sucrose octasulfate, caractérisée en ce que ladite composition est un mélange physique et en ce que le ratio massique entre la teneur en acide hyaluronique [HA] et la teneur en sel hydrosoluble de sucrose octasulfate [SOS], [HA]/[SOS] est supérieur ou égal à 0,1.

On entend par acide hyaluronique, l'acide hyaluronique, réticulé ou non réticulé, seul ou en mélange, éventuellement modifié chimiquement par substitution, seul ou en mélange, éventuellement sous forme de l'un de ses sels, seul ou en mélange.

Dans un mode de réalisation, la composition selon l'invention comprend au moins un acide hyaluronique non réticulé ou l'un de ses sels, seul ou en mélange.

Dans un mode de réalisation, la composition selon l'invention comprend au moins un acide hyaluronique réticulé ou l'un de ses sels, seul ou en mélange.

Dans un mode de réalisation, la composition selon l'invention comprend au moins un acide hyaluronique coréticulé ou l'un de ses sels, seul ou en mélange.

Dans un mode de réalisation, la composition selon l'invention comprend au moins un acide hyaluronique modifié chimiquement par substitution, réticulé ou non réticulé, ou l'un de ses sels, seul ou en mélange.

Dans un mode de réalisation, l'acide hyaluronique est sous forme de sel de sodium ou de potassium.

Dans un mode de réalisation, la composition selon l'invention est caractérisée en ce que le sel hydrosoluble de sucrose octasulfate est choisi dans le groupe constitué par les sels de métaux alcalins, les sels de métaux alcalino-terreux, les sels d'argent, les sels d'ammonium, les sels d'acides aminés.

Dans un mode de réalisation, la composition selon l'invention est caractérisée en ce que le sel hydrosoluble de sucrose octasulfate est choisi dans le groupe constitué par les sels de métaux alcalins ou les sels de métaux alcalino-terreux.

Dans un mode de réalisation, la composition selon l'invention est caractérisée en ce que le sel hydrosoluble de sucrose octasulfate est le sel de sodium de sucrose octasulfate ou le sel de potassium de sucrose octasulfate.

Dans un mode de réalisation, la composition selon l'invention est caractérisée en ce que le sel hydrosoluble de sucrose octasulfate est le sel de sodium de sucrose octasulfate.

Dans un mode de réalisation, la composition selon l'invention est caractérisée en ce que le sel hydrosoluble de sucrose octasulfate est le sel de potassium de sucrose octasulfate.

La solubilité maximale du sel de potassium de sucrose octasulfate est de 40 mg/g, ainsi le ratio [HA]/[SOS] minimum dans les compositions de la présente invention est de 0,1, étant donné que la concentration minimale acceptable en HA dans les compositions de la présente invention est de 4 mg/g afin d'obtenir une consistance adéquate avec les utilisations de la présente invention. La solubilité du sel de sodium de sucrose octasulfate étant supérieure à celle du sel de potassium, la limite de solubilité du sel de potassium de sucrose octasulfate a été prise en considération pour déterminer la valeur minimum du ratio [HA]/[SOS].

On entend par milieu aqueux, une composition sous forme de solution aqueuse, éventuellement en présence de sel soluble ou de tampon, éventuellement sous forme de gel ou hydrogel.

On entend par mélange physique, un mélange dans lequel l'intégrité des propriétés physicochimiques des constituants est conservée, c'est-à-dire qu'elles sont strictement identiques à celles des constituants du mélange pris séparément, cette intégrité des propriétés physicochimiques pouvant être caractérisée par différentes méthodes analytiques. La demanderesse a choisi pour caractériser le mélange physique la spectroscopie par résonance magnétique nucléaire (RMN). Le spectre RMN du mélange selon l'invention est un spectre à 1 dimension du proton $^1H$. Par exemple, le spectre RMN d'un mélange physique comprenant un sel hydrosoluble de sucrose octasulfate est caractérisé en ce que les valeurs des déplacements chimiques des protons du sel hydrosoluble de sucrose octasulfate en solution, seul, sont égales aux valeurs des déplacements chimiques des protons du sel hydrosoluble de sucrose octasulfate dans le mélange physique selon l'invention, ce qui prouve qu'il n'y a pas d'interaction ou de réaction entre le sel hydrosoluble de sucrose octasulfate et l'acide hyaluronique.

Dans un mode de réalisation, la composition selon l'invention est caractérisée en ce que, lorsqu'elle est analysée par spectroscopie RMN du proton $^1H$, la valeur du déplacement chimique du proton anomérique ($\delta$=5,7 ppm) du sel de sucrose octasulfate est identique à la valeur du déplacement chimique du proton anomérique du sel de sucrose octasulfate seul en solution.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 0,1 et 5000.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 0,1 et 2500.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 1 et 1000.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 10 et 500.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 0,1 et 100.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est compris entre 0,5 et 50.

Dans un mode de réalisation, la composition selon l'invention est caractérisée en ce que le ratio massique entre la teneur en acide hyaluronique [HA] et la teneur en sel hydrosoluble de sucrose octasulfate [SOS], [HA]/[SOS] est égal à 20.

Dans un mode de réalisation, la composition selon l'invention est caractérisée en ce que la teneur en sel hydrosoluble de sucrose octasulfate est comprise entre 0,01 mg/g et 40 mg/g de composition.

Dans un mode de réalisation, la composition selon l'invention est caractérisée en ce que la teneur en sel hydrosoluble de sucrose octasulfate est comprise entre 0,1 mg/g et 10 mg/g de composition.

Dans un mode de réalisation, la composition selon l'invention est caractérisée en ce que la teneur en sel hydrosoluble de sucrose octasulfate est comprise entre 0,1 mg/g et 1 mg/g de composition.

Dans un mode de réalisation, la composition selon l'invention est caractérisée en ce que la teneur en acide hyaluronique est comprise entre 2 mg/g et 50 mg/g de composition.

Dans un mode de réalisation, la composition selon l'invention est caractérisée en ce que la teneur en acide hyaluronique est comprise entre 4 mg/g et 40 mg/g de composition.

Dans un mode de réalisation, la composition selon l'invention est caractérisée en ce que la teneur en acide hyaluronique est comprise entre 10 mg/g et 30 mg/g de composition.

Dans un mode de réalisation, la composition selon l'invention est caractérisée en ce qu'elle comprend au moins un acide hyaluronique non réticulé.

Dans un mode de réalisation, la composition selon l'invention est caractérisée en ce qu'elle comprend au moins un acide hyaluronique réticulé.

Dans la présente invention, le taux de réticulation X, est défini comme étant égal au rapport :

$$X = \frac{\text{(Nombre de moles de réticulant introduites dans le milieu réactionnel)}}{\text{(Nombre de moles de motif disacharidique introduites dans le milieu réactionnel)}}$$

Dans un mode de réalisation, l'acide hyaluronique réticulé présente un taux de réticulation X compris entre 0,001 et 0,5.

Dans un mode de réalisation, l'acide hyaluronique réticulé présente un taux de réticulation X compris entre 0,01 et 0,25.

Dans un mode de réalisation, l'acide hyaluronique réticulé présente un taux de réticulation X compris entre 0,1 et 0,2.

Dans un mode de réalisation, l'acide hyaluronique est co-réticulé tel que décrit dans la demande WO 2000/0046253 au nom de Fermentech ou WO 2004/092222 au nom de Cornéal ou même FR2865737 au nom de ANTEIS.

Dans un mode de réalisation, la composition selon l'invention est caractérisée en ce qu'elle comprend un mélange d'acides hyaluroniques réticulé et non réticulé tel que décrit dans la demande WO2005061611 au nom de Innomed Ltd.

Dans un mode de réalisation, la composition selon l'invention est caractérisée en ce qu'elle comprend un mélange d'acides hyaluroniques réticulés.

Dans un mode de réalisation, le mélange d'acides hyaluroniques réticulés est un mélange monophasique tel que celui décrit dans la demande de brevet WO 2009/071697 au nom de la demanderesse.

Dans un mode de réalisation, la composition selon l'invention est caractérisée en ce qu'elle comprend au moins un acide hyaluronique substitué par un groupement apportant des propriétés lipophile ou hydratante, comme par exemple les acides hyaluroniques substitués tels que décrits dans la demande WO2013079889 au nom de la demanderesse.

Dans un mode de réalisation, la composition comprend, en outre, un autre polysaccharide.

Dans un mode de réalisation cet autre polysaccharide est choisi dans le groupe constitué par la cellulose, l'acide alginique ou l'un de leurs sels.

On appelle Mw ou « masse moléculaire », la masse moléculaire moyenne en masse des polymères, mesurée en Daltons.

Dans un mode de réalisation, la composition selon l'invention est caractérisée en ce que la masse moléculaire Mw de l'acide hyaluronique est comprise dans un intervalle de 0,01 MDa et 5 MDa.

Dans un mode de réalisation, la composition selon l'invention est caractérisée en ce que la masse moléculaire Mw de l'acide hyaluronique est comprise dans un intervalle de 0,1 MDa et 3,5 MDa.

Dans un mode de réalisation, la composition selon l'invention est caractérisée en ce qu'elle comprend, en outre, au moins un principe actif.

Dans un mode de réalisation, la composition selon l'invention est caractérisée en ce que le principe actif est choisi parmi les antioxydants, les anesthésiques locaux, les vitamines, seuls ou en combinaison.

Dans un mode de réalisation, la composition selon l'invention est caractérisée en ce que les antioxydants sont choisis parmi les polyols.

Dans un mode de réalisation, la composition selon l'invention est caractérisée en ce que les antioxydants sont choisis parmi le mannitol et le sorbitol, seul ou en combinaison.

Dans un mode de réalisation, la composition selon l'invention est caractérisée en ce que l'agent antioxydant est le mannitol.

Dans un mode de réalisation, la composition selon l'invention est caractérisée en ce que l'agent antioxydant est le sorbitol.

Dans un mode de réalisation, la composition selon l'invention est caractérisée en ce que l'agent antioxydant est une combinaison de mannitol et de sorbitol.

Dans un mode de réalisation, la composition selon l'invention est caractérisée en ce que les anesthésiques locaux sont choisis dans le groupe constitué par la lidocaïne, la procaïne, la mépivacaïne, la ropivacaïne, la bupivacaïne, et leurs sels pharmaceutiquement acceptables.

Dans un mode de réalisation, la composition selon l'invention est caractérisée en ce que l'anesthésique local est le chlorhydrate de lidocaïne.

Dans un mode de réalisation, la composition selon l'invention est caractérisée en ce que la teneur en principe(s) actif(s) est comprise entre 0,01 et 10 % en poids par rapport au poids total de la composition.

Dans un mode de réalisation, la composition selon l'invention est caractérisée en ce que la teneur en principe(s) actif(s) est comprise entre 0,1 et 5 % en poids par rapport au poids total de la composition.

Dans un mode de réalisation, la composition selon l'invention est caractérisée en ce qu'elle est stable à la stérilisation par autoclavage à la vapeur.

Dans un mode de réalisation, l'invention est une composition de viscosupplémentation caractérisée en ce qu'elle comprend au moins une composition selon l'invention.

Dans un mode de réalisation, l'invention est une composition pour le traitement de la xérophtalmie ou sécheresse oculaire, caractérisée en ce qu'elle comprend au moins une composition selon l'invention.

Selon les modes de réalisation, lesdites compositions sont utilisées en tant que larmes artificielles, gels lacrymaux ou lubrifiants.

Dans un mode de réalisation, l'invention est une formulation cosmétique caractérisée en ce qu'elle comprend une composition selon l'invention et au moins un excipient cosmétiquement acceptable.

Dans un mode de réalisation, la formulation cosmétique selon l'invention est caractérisée en ce qu'elle comprend entre 0,01 et 10 % en poids d'une composition selon l'invention par rapport au poids total de ladite formulation cosmétique.

Dans un mode de réalisation, la formulation cosmétique selon l'invention est caractérisée en ce que le au moins un excipient cosmétiquement acceptable est choisi parmi les adjuvants cosmétiques classiques.

On entend par adjuvants cosmétiques classiques, des excipients notamment choisis parmi les corps gras, les solvants organiques, les épaississants ioniques ou non ioniques, les adoucissants, les antioxydants, les opacifiants, les stabilisants, les émollients, les silicones, les $\alpha$-hydroxyacides, les agents anti-mousse, les agents hydratants, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique.

La formulation cosmétique selon l'invention peut comprendre en outre d'autres agents actifs, notamment des agents hydratants, humectants, apaisants, anti-inflammatoires, cicatrisants.

Les formulations cosmétiques selon l'invention peuvent être préparées selon les techniques bien connues de l'homme de l'art, en particulier celles destinées à la préparation d'émulsions de type Huile dans Eau (H/E) ou Eau dans Huile (E/H).

Cette formulation cosmétique peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Dans un mode de réalisation, la formulation cosmétique est stérilisée selon les techniques bien connues de l'homme de l'art, et en particulier par irradiation gamma.

La présente invention concerne également une formulation cosmétique selon l'invention caractérisée en ce qu'elle est administrable par voie topique sous forme de lotion, crème, huile, stick, shampooing ou toutes autres formes appropriées.

La formulation cosmétique, dermatologique ou pharmaceutique selon l'invention peut être utilisée comme composition protectrice de l'épiderme humain contre les agressions des éléments extérieurs et ainsi lutter contre le vieillissement prématuré de l'épiderme.

L'invention concerne également un procédé de fabrication d'une composition selon l'invention.

Dans un mode de réalisation le procédé selon l'invention est caractérisé en ce qu'il comprend au moins :

- Une étape d'hydratation des fibres d'au moins un acide hyaluronique, pour obtenir un hydrogel,
- Une étape de mélange d'une solution de sel hydrosoluble de sucrose octasulfate avec l'hydrogel obtenu à l'étape précédente,
- Une étape d'homogénéisation, et
- Une étape d'autoclavage à la vapeur.

Dans un mode de réalisation, le procédé selon l'invention est caractérisé en ce que l'étape d'hydratation est

réalisée à température ambiante.

Dans un mode de réalisation, le procédé selon l'invention est caractérisé en ce que l'étape d'homogénéisation est réalisée à température ambiante.

Dans un mode de réalisation, le procédé selon l'invention est caractérisé en ce que l'étape d'autoclavage à la vapeur est réalisée à une température de 121 à 134°C, pendant une durée adaptée à la température.

Dans un mode de réalisation, le procédé selon l'invention est caractérisé en ce qu'il comprend en outre au moins une étape de conditionnement du mélange homogénéisé dans des seringues.

Dans un mode de réalisation, le procédé selon l'invention est caractérisé en ce qu'il comprend en outre au moins une étape de conditionnement du mélange homogénéisé dans des flacons uni-doses.

Dans un mode de réalisation, le procédé selon l'invention est caractérisé en ce qu'il comprend en outre au moins une étape de réticulation.

Dans un mode de réalisation, le procédé selon l'invention est caractérisé en ce que l'étape de réticulation se situe entre l'étape d'hydratation et l'étape de mélange.

Dans un mode de réalisation, le procédé selon l'invention est caractérisé en ce qu'il comprend une étape de mélange de deux acides hyaluroniques préalablement réticulés comme dans le procédé décrit dans WO2013/079889.

Dans un mode de réalisation, le procédé selon l'invention est caractérisé en ce que l'étape de réticulation est réalisée au moyen d'au moins un agent de réticulation.

Dans un mode de réalisation, le procédé selon l'invention est caractérisé en ce que l'agent de réticulation est bi- ou polyfonctionnel.

Dans un mode de réalisation, le procédé selon l'invention est caractérisé en ce que l'agent de réticulation bi- ou polyfonctionnel est choisi parmi le groupe constitué par l'éther d'éthylèneglycoldiglycidyle, l'éther de butanedioldiglycidyle (BDDE), l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde, l'épichlorohydrine ou bien encore le glutaraldéhyde, les carbodiimides tels que par exemple le 1-éthyl-3-[3-dimethylaminopropyl]carbodiimide hydrochloride (EDC).

Dans un mode de réalisation, le procédé selon l'invention est caractérisé en ce que l'agent de réticulation bifonctionnel est l'éther de butanedioldiglycidyle (BDDE) ou le 1,2,7,8-diépoxyoctane.

Dans un mode de réalisation, le procédé de fabrication selon l'invention est caractérisé en ce que l'étape de réticulation est mise en oeuvre selon les techniques connues de l'homme du métier.

Dans un mode de réalisation, le procédé selon l'invention est caractérisé en ce qu'il comprend après l'étape de réticulation, au moins une étape de purification et lavage mise en oeuvre selon les techniques connues de l'homme du métier.

L'invention concerne l'utilisation d'une composition selon l'invention, pour la formulation d'une composition de viscosupplémentation.

L'invention concerne l'utilisation d'une composition selon l'invention, pour la formulation d'une composition pour le comblement de rides.

Les applications visées sont plus particulièrement les applications communément répandues dans le cadre des viscoélastiques injectables et des polysaccharides utilisés ou potentiellement utilisables dans les pathologies ou traitements suivants :

- Injections esthétiques au niveau du visage : de comblement de rides, défauts cutanés ou volumatrices (pommettes, menton, lèvres) ;
- Injections volumatrices au niveau du corps : augmentation des seins et des fesses, augmentation du point G, vaginoplastie, reconstruction des lèvres vaginales, augmentation du pénis ;
- Traitement de l'arthrose, injection dans l'articulation en remplacement ou en complément du liquide synovial déficient ;
- Injection péri-urétrale pour le traitement de l'incontinence urinaire par insuffisance sphinctérienne ;
- Injection post-chirurgicale pour éviter les adhésions péritonéales notamment ;
- Injection suite à une chirurgie de la presbytie par incisions sclérales au laser ;
- Injection dans la cavité vitréenne.

Plus particulièrement, en chirurgie esthétique, en fonction de ses propriétés viscoélastiques et de rémanence, l'hydrogel obtenu selon le procédé de l'invention pourra être utilisé :

- pour le comblement des rides fines, moyennes ou profondes, et être injecté avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien

à l'emplacement de l'injection.

La composition selon l'invention trouve également une application importante en chirurgie articulaire et en chirurgie dentaire pour le comblement des poches parodontales par exemple.

Ces exemples d'utilisation ne sont nullement limitatifs, la composition selon la présente invention étant plus largement prévue pour :

- combler des volumes ;
- générer des espaces au sein de certains tissus, favorisant ainsi leur fonctionnement optimal ;
- remplacer des liquides physiologiques déficients.

L'invention concerne également un kit comprenant une composition selon l'invention, conditionnée en seringues et stérilisée après conditionnement.

L'invention concerne également un kit comprenant une composition selon l'invention, conditionnée en flacons uni-doses et stérilisée après conditionnement.

La composition selon l'invention trouve également une application dans le domaine cosmétique ou pharmaceutique.

Plus particulièrement dans le domaine cosmétique, une composition selon l'invention sera utilisée comme ingrédient actif hydratant dans une composition cosmétique.

Dans le domaine pharmaceutique, une composition selon l'invention sera utilisée comme composition d'hydratation des yeux atteints de sécheresse oculaire, à savoir comme larme artificielle.

Dans ces applications cosmétiques et/ou pharmaceutiques, les compositions peuvent comprendre en outre tout ingrédient cosmétiquement ou pharmaceutiquement acceptable.

Les caractéristiques de la composition selon la présente invention ainsi que ses procédés de fabrication et leurs propriétés sont illustrés dans les exemples ci-après.

EXEMPLE 1 :

Cet exemple illustre une composition selon l'invention comprenant de l'acide hyaluronique non réticulé et du sucrose octasulfate de potassium.

Des fibres de hyaluronate de sodium (NaHA) de qualité injectable (1 g ; masse moléculaire : environ 2,7 MDa) sont pesées dans un récipient. Une solution aqueuse de tampon phosphate (32,3 g) est ajoutée, le tout est homogénéisé pendant environ 1 heure à la spatule, à température ambiante et sous une pression atmosphérique de 900 mmHg.

L'hydrogel de NaHA non réticulé ainsi obtenu à une concentration d'environ 30 mg/g en NaHA.

Du sucrose octasulfate de potassium (KSOS) (60 mg soit $4,7.10^{-5}$ mol) est solubilisé dans une solution de tampon phosphate (19,94 g) pour obtenir une solution aqueuse de sucrose octasulfate de potassium de concentration 3 mg/g.

L'hydrogel de NaHA obtenu à l'étape précédente est dilué par ajout de la solution aqueuse de sucrose octasulfate de potassium précédemment préparée. La composition ainsi obtenue est ensuite homogénéisée.

On obtient ainsi une composition comprenant du NaHA non réticulé à une concentration de 20 mg/g et du KSOS à une concentration de 1 mg/g ; le ratio massique [HA]/[SOS] est donc de 20.

La composition ainsi obtenue est conditionnée en seringues qui sont stérilisées par autoclavage à la vapeur (T= 121°C, 10 min).

Selon un mode opératoire identique à celui décrit ci-dessus en adaptant les quantités mises en oeuvre sont préparés les gels décrits dans le tableau 7.

Exemple 2 :

Cet exemple illustre un exemple de composition selon l'invention comprenant de l'acide hyaluronique réticulé et du sucrose octasulfate de potassium.

La composition comprenant de l'acide hyaluronique réticulé est obtenue selon le mode opératoire de réticulation décrit dans WO 2009/071697 (exemple 1, première partie) au nom de VIVACY à partir de fibres de hyaluronate de sodium (NaHA) (1g ; masse moléculaire : environ 2,7 MDa) et de l'éther de butanedioldiglycidyle (BDDE) (54 mg). La composition ainsi obtenue a une concentration d'environ 30 mg/g en NaHA réticulé avec un taux de réticulation X d'environ 0,12.

Une solution aqueuse de sucrose octasulfate de potassium de concentration 3 mg/g est préparée comme à l'exemple 1.

L'hydrogel de NaHA réticulé obtenu à l'étape précédente est dilué dans la solution aqueuse de sucrose octasulfate de potassium précédemment préparée. La composition ainsi obtenue est ensuite homogénéisée.

On obtient ainsi une composition comprenant du NaHA réticulé à une concentration de 20 mg/g et du sucrose octasulfate de potassium à une concentration de 1 mg/g ; le ratio massique [HA]/[SOS] est donc de 20.

La composition ainsi obtenue est conditionnée en seringues qui sont stérilisées par autoclavage à la vapeur (T=121°C, 10 min).

Selon un mode opératoire identique à celui décrit ci-dessus en adaptant les quantités mises en oeuvre est préparé le gel décrit dans le tableau 8.

EXEMPLE 3

Cet exemple illustre un exemple de composition selon l'invention comprenant de l'acide hyaluronique non réticulé, du sucrose octasulfate de potassium et du mannitol.

La composition comprenant de l'acide hyaluronique non réticulé et du sucrose octasulfate de potassium est préparée selon le mode opératoire de l'exemple 1, à partir d'un hydrogel d'acide hyaluronique à une concentration de 30 mg/g et d'une solution de sucrose octasulfate de potassium à une concentration de 10 mg/g.

L'addition d'une solution de mannitol à une concentration de 86 mg/g à la composition obtenue ci-dessus est effectuée selon le mode opératoire décrit dans WO 2009/024670 au nom de ANTEIS.

La composition ainsi obtenue comprend de l'acide hyaluronique non réticulé à une concentration de 20 mg/g, du mannitol à une concentration de 20 mg/g et du sucrose octasulfate de potassium à une concentration de 1 mg/g ; le ratio massique [HA]/[SOS] est donc de 20.

La composition ainsi obtenue est conditionnée en seringues qui sont stérilisées par autoclavage à la vapeur (T=121°C, 10 min).

EXEMPLE 4

Cet exemple illustre un exemple de composition selon l'invention comprenant de l'acide hyaluronique réticulé, du sucrose octasulfate de potassium et du mannitol.

Une composition comprenant du NaHA réticulé est préparée selon le mode opératoire décrit à l'exemple 2 à partir d'un hydrogel de NaHA à une concentration de 30 mg/g et du sucrose octasulfate de potassium à une concentration de 10 mg/g.

L'addition d'une solution de mannitol à une concentration de 86 mg/g à la composition obtenue ci-dessus est effectuée selon le mode opératoire décrit dans WO 2009/024670 au nom de ANTEIS.

La composition ainsi obtenue comprend de l'acide hyaluronique réticulé à une concentration de 20 mg/g, du mannitol à une concentration de 20 mg/g et du sucrose octasulfate de potassium à une concentration de 1 mg/g ; le ratio massique [HA]/[SOS] est donc de 20.

La composition ainsi obtenue est conditionnée en seringues qui sont stérilisées par autoclavage à la vapeur (T=121°C, 10 min).

EXEMPLE 5

Cet exemple illustre un exemple de composition selon l'invention comprenant de l'acide hyaluronique non réticulé, du sucrose octasulfate de potassium et de la lidocaïne.

La composition comprenant de l'acide hyaluronique non réticulé et du sucrose octasulfate de potassium est préparée selon le mode opératoire de l'exemple 1, à partir d'un hydrogel d'acide hyaluronique à une concentration de 30 mg/g et d'une solution de sucrose octasulfate de potassium à une concentration de 10 mg/g.

L'addition d'une solution de lidocaïne à une concentration de 13 mg/g à la composition obtenue ci-dessus est effectuée selon le mode opératoire décrit dans WO 2009/024670 au nom de ANTEIS ou selon le mode opératoire décrit dans les demandes US 61/791,977 ou FR 13/52971 au nom de VIVACY.

La composition ainsi obtenue comprend de l'acide hyaluronique non réticulé à une concentration de 20 mg/g, de la lidocaïne à une concentration de 3 mg/g et du sucrose octasulfate de potassium à une concentration de 1 mg/g ; le ratio massique [HA]/[SOS] est donc de 20.

La composition ainsi obtenue est conditionnée en seringues qui sont stérilisées par autoclavage à la vapeur (T=121°C, 10 min).

EXEMPLE 6

Cet exemple illustre un exemple de composition selon l'invention comprenant de l'acide hyaluronique réticulé, du sucrose octasulfate de potassium et de la lidocaïne.

Une composition comprenant du NaHA réticulé est préparée selon le mode opératoire décrit à l'exemple 2 à partir d'un hydrogel de NaHA à une concentration de 30 mg/g et du sucrose octasulfate de potassium à une concentration de 10 mg/g.

L'addition d'une solution de lidocaïne à une concentration de 13 mg/g à la composition obtenue ci-dessus est effectuée selon le mode opératoire décrit dans WO 2009/024670 au nom de ANTEIS ou selon le mode opératoire décrit dans les demandes US 61/791,977 ou FR 13/52971 au nom de VIVACY.

La composition ainsi obtenue comprend de l'acide hyaluronique réticulé à une concentration de 20 mg/g, de la lidocaïne à une concentration de 3 mg/g et du sucrose octasulfate de potassium à une concentration de 1 mg/g ; le ratio massique [HA]/[SOS] est donc de 20.

La composition ainsi obtenue est conditionnée en seringues qui sont stérilisées par autoclavage à la vapeur (T=121°C, 10 min).

Les compositions obtenues aux exemples 1 à 6 peuvent être utilisées en viscosupplémentation ou en fluide de comblement.

EXEMPLE 7

Cet exemple illustre une composition selon l'invention comprenant de l'acide hyaluronique non réticulé et du sucrose octasulfate de potassium pour le traitement de la sécheresse oculaire.

Des fibres de hyaluronate de sodium (NaHA) de qualité injectable (1 g ; masse moléculaire : environ 1 MDa) sont pesées dans un récipient. Une solution aqueuse de tampon phosphate (165,7 g) est ajoutée, le tout est homogénéisé pendant environ 1 heure à la spatule, à température ambiante et sous une pression atmosphérique de 900 mmHg.

L'hydrogel de NaHA non réticulé ainsi obtenu a une concentration d'environ 6 mg/g en NaHA.

Du sucrose octasulfate de potassium (KSOS) (600 mg soit 4,7.10-4 mol) est solubilisé dans une solution de tampon phosphate (19,94 g) pour obtenir une solution aqueuse de sucrose octasulfate de potassium de concentration 30 mg/g.

L'hydrogel de NaHA obtenu à l'étape précédente est dilué par ajout de la solution aqueuse de sucrose octasulfate de potassium précédemment préparée. La composition ainsi obtenue est ensuite homogénéisée.

On obtient ainsi une composition comprenant du NaHA non réticulé à une concentration de 4 mg/g et du KSOS à une concentration de 10 mg/g ; le ratio massique [HA]/[SOS] est donc de 0,4.

La composition ainsi obtenue est conditionnée en flacons uni-doses, qui sont stérilisés.

EXEMPLE 8

Cet exemple illustre une formulation cosmétique.

| | |
|---|---|
| Alcool cétéarylique | 5 % |
| Esters (éther dicaprylique, myristate de myristyle) | 10 % |
| Composition acide hyaluronique non réticulé, sucrose octasulfate de potassium obtenue à l'exemple 1 | 1 % |
| Glycérine | 5 % |
| Alcool | 5 % |
| Tocophérol | 0,2 % |
| Parfum | 0,1 % |
| Eau | qsp 100% |

EXEMPLE 9

Cet exemple illustre une formulation cosmétique stérilisée.

| | |
|---|---|
| Composition acide hyaluronique non réticulé, sucrose octasulfate de potassium obtenue selon l'exemple 1 avec un ratio [HA]/[SOS] de 4 | 1 % |
| Carbomer | 0,5 % |
| Gomme Xanthane | 2,5 % |
| Acide citrique | 0,1 % |
| 2-phényléthanol | 0,5 % |
| Glycérine | 1,2 % |
| Eau | qsp 100% |

La formulation cosmétique est stérilisée par irradiation gamma à des doses de 5-25 kGy et de préférence de 7-15 kGy.

EXEMPLE 10

Stabilité à l'autoclavage à la vapeur.

Pour les compositions selon l'invention comprenant de l'acide hyaluronique non réticulé, la viscosité η des compositions stérilisées après l'étape d'autoclavage à la vapeur est caractérisée sur rhéomètre TA Instruments AR 2000 Ex, en contrainte imposée à 25°C. La valeur de viscosité est relevée à une contrainte de 0,02 $s^{-1}$.

Pour les compositions comprenant de l'acide hyaluronique non réticulé, le pourcentage de perte de la viscosité est la valeur de la différence de la viscosité de la composition de référence après autoclavage à la vapeur et la viscosité de la composition comprenant de l'acide hyaluronique non réticulé et un oligo-polysaccharide ou un ose sulfaté après autoclavage à la vapeur sur la valeur de la viscosité de la composition de référence après autoclavage à la vapeur.

Pour les compositions selon l'invention comprenant de l'acide hyaluronique réticulé, la composante élastique G' des compositions stérilisées après l'étape d'autoclavage est caractérisée sur rhéomètre TA Instruments AR 2000 Ex, en oscillation à 25°C, les valeurs de la composante élastique étant relevées à une fréquence de 1 Hz.

Pour les compositions comprenant de l'acide hyaluronique réticulé, le pourcentage de perte de la composante élastique G' est la valeur de la différence de la composante élastique G' de la composition de référence après autoclavage à la vapeur et la composante élastique G' de la composition comprenant de l'acide hyaluronique réticulé et un oligo-polysaccharide ou un ose sulfaté après autoclavage à la vapeur sur la valeur de la composante élastique G' de la composition de référence après autoclavage à la vapeur.

Pour toutes ces mesures une composition de référence est formulée, en remplaçant la solution aqueuse d'oligosaccharide ou d'ose sulfaté par une même quantité de solution aqueuse de tampon phosphate.

a) Stabilité à l'autoclavage à la vapeur de compositions comprenant de l'acide hyaluronique non réticulé et un oligo-polysaccharide sulfaté ou un ose sulfaté

Neuf compositions comprenant de l'acide hyaluronique non réticulé et un oligo-polysaccharide sulfaté ou un ose sulfaté à une concentration de 1 mg/g dans la composition sont préparées selon le mode opératoire décrit à l'exemple 1.

Une composition de référence est également formulée, en remplaçant la solution aqueuse d'oligo-polysaccharide ou d'ose sulfaté par une même quantité de solution aqueuse de tampon phosphate.

Les pourcentages de perte de la viscosité des compositions selon le mode opératoire de l'exemple 1 par rapport à la composition de référence après l'étape d'autoclavage à la vapeur sont mesurés, et les résultats obtenus sont présentés dans le tableau 1 ci-dessous :

Tableau 1

| Oligo-polysaccharides et oses sulfatés | % de perte de la viscosité par rapport à la composition de référence |
|---|---|
| N-Acetyl-D-Glucosamine-6-Sulfate | 96 |
| D-Glucosamine-6-Sulfate | 98 |
| D-Glucosamine-2-Sulfate | 87 |
| Dextrane Sulfate $_{(40000)}$ | 16 |
| Chondroïtine Sulfate | 62 |
| Carragénane$_{(disaccharide)}$ diSulfaté | 94 |
| Carragénane$_{(hexasaccharide)}$ hexasulfaté | 92 |
| Sucrose octasulfate de sodium | 0 |
| Sucrose octasulfate de potassium | 0 |

Pourcentage de perte de la viscosité après l'étape d'autoclavage à la vapeur de compositions comprenant de l'acide hyaluronique non réticulé et un oligo-polysaccharide sulfaté ou un ose sulfaté à une concentration de 1 mg/g par rapport à la composition de référence

On observe, après l'étape d'autoclavage à la vapeur, une perte de la viscosité pour toutes les compositions à l'exception de celles comprenant un sel hydrosoluble de sucrose octasulfate. Les pertes les plus importantes sont

observées pour les compositions comprenant des glucosamines sulfatées (N-acétylées ou non et sulfatées en position 2 ou 6). S'agissant de celles comprenant un oligo-polysaccharide sulfaté tel que le dextrane sulfate ou la chondroïtine sulfate elles apparaissent moins importantes.

b) Stabilité à l'autoclavage à la vapeur de compositions comprenant de l'acide hyaluronique non réticulé, un oligo-polysaccharide sulfaté ou un ose sulfaté et du mannitol

Une série de cinq compositions comprenant de l'acide hyaluronique non réticulé, du mannitol et un oligo-polysaccharide sulfaté ou un ose sulfaté à une concentration de 1 mg/g dans la composition est préparée selon le mode opératoire décrit à l'exemple 3.
Une composition de référence est également formulée, en remplaçant la solution aqueuse d'oligo-polysaccharide ou d'ose sulfaté par une même quantité de solution aqueuse de tampon phosphate.
Les pourcentages de perte de la viscosité des compositions selon le mode opératoire de l'exemple 3 par rapport à la composition de référence sont mesurés et les résultats obtenus sont présentés dans le tableau 2 ci-dessous :

Tableau 2

| Oligo-polysaccharides et oses sulfatés | % de perte de la viscosité par rapport à la composition de référence |
|---|---|
| N-Acetyl-D-Glucosamine-6-Sulfate | 60 |
| D-Glucosamine-6-Sulfate | 90 |
| D-Glucosamine-2-Sulfate | 25 |
| Sucrose octasulfate de sodium | 0 |
| Sucrose octasulfate de potassium | 0 |

Pourcentage de perte de la viscosité après l'étape d'autoclavage à la vapeur de compositions comprenant de l'acide hyaluronique non réticulé, du mannitol et un oligo-polysaccharide sulfaté ou un ose sulfaté à une concentration de 1 mg/g par rapport à la composition de référence
On observe pour les compositions comprenant de l'acide hyaluronique non réticulé, du mannitol et une glucosamine sulfate une perte de la viscosité par rapport à la composition de référence.
Les seules compositions pour lesquelles on n'observe aucune perte de la viscosité après l'autoclavage à la vapeur par rapport à la composition de référence sont les compositions comprenant un sel hydrosoluble de sucrose octasulfate.

c) Stabilité à l'autoclavage à la vapeur de compositions comprenant de l'acide hyaluronique réticulé, un oligo-polysaccharide sulfaté ou un ose sulfaté et du mannitol

Une série de quatre compositions comprenant de l'acide hyaluronique réticulé, du mannitol et un oligo-polysaccharide sulfaté ou un ose sulfaté à une concentration de 1 mg/g dans la composition est préparée selon le mode opératoire décrit à l'exemple 4.
Une composition de référence est également formulée, en remplaçant la solution aqueuse d'oligo-polysaccharide ou d'ose sulfaté par une quantité équivalente de solution aqueuse de tampon phosphate.
Les pourcentages de perte de la composante élastique G' des compositions selon le mode opératoire de l'exemple 4 par rapport à la composition de référence sont mesurés et les résultats obtenus sont présentés dans le tableau 3 ci-dessous :

Tableau 3

| Oligo-polysaccharides et oses sulfatés | % de perte de la composante élastique G' par rapport à la composition de référence |
|---|---|
| N-Acetyl-D-Glucosamine-6-Sulfate | 32 |
| D-Glucosamine-6-Sulfate | 74 |
| D-Glucosamine-2-Sulfate | 18 |
| Sucrose octasulfate de potassium | 0 |

Pourcentage de perte de la composante élastique G' après l'étape d'autoclavage à la vapeur de compositions comprenant de l'acide hyaluronique réticulé, du mannitol, et un oligo-polysaccharide sulfaté ou un ose sulfaté à une concentration de 1 mg/g par rapport à la composition de référence

On observe des pertes de la composante élastique G' par rapport à la composition de référence pour les compositions comprenant des glucosamines sulfatées (N-acétylées ou non et sulfatées en position 2 ou 6).

On observe pour les compositions comprenant du sucrose octasulfate de potassium qu'il n'y a aucune perte de la composante G' après autoclavage à la vapeur par rapport à la composition de référence.

En conclusion, on n'observe aucune perte de la viscosité ou de la composante élastique G' après l'autoclavage à la vapeur par rapport à la composition de référence seulement pour les compositions comprenant des sels hydrosolubles de sucrose octasulfate de sodium ou de potassium. Cette observation est la même que l'acide hyaluronique soit ou non réticulé et que les compositions comprennent ou pas du mannitol.

Par conséquent, ces compositions sont les seules qui permettent d'obtenir des produits tout aussi stables que la composition de référence après l'autoclavage à la vapeur.

EXEMPLE 11

Caractéristiques spectroscopiques du mélange physique comprenant de l'acide hyaluronique et du sucrose octasulfate de potassium

Une analyse RMN 1H a été réalisée sur la composition de l'exemple 4. Les caractérisations spectroscopiques ont été effectuées sur la composition de l'exemple 4 avant stérilisation et après stérilisation par autoclavage à la vapeur.

Les analyses en solution sont réalisées sur un spectromètre Bruker Avance opérant à 600 MHz (1H) et équipé d'une sonde TCI (cryosonde).

Les résultats sont présentés ci-dessous :

- La figure 1 est le spectre RMN 1H du sucrose octasulfate de potassium seul en solution dans l'eau.
- La figure 2 est une superposition des spectres RMN 1H de la composition de l'exemple 4 avant et après stérilisation.

Le spectre RMN 1H de la figure 1 du sucrose octasulfate de potassium seul en solution dans l'eau a permis l'attribution partielle des signaux. On prend comme référence la valeur du déplacement chimique du proton anomérique du sucrose octasulfate de potassium qui est de $\delta$ = 5,7 ppm.

Représentation du proton anomérique du sucrose octasulfate

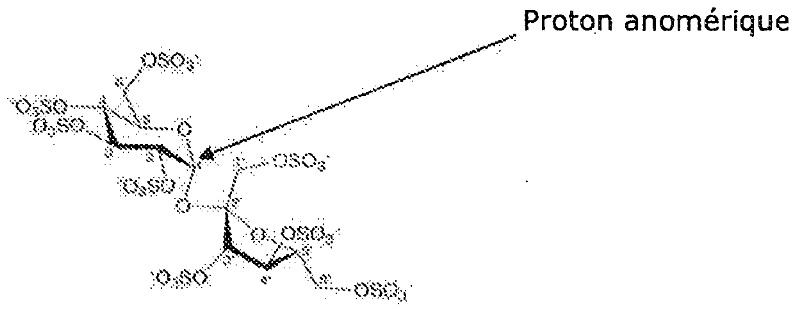


Sucrose Octasulfate

Les valeurs du déplacement chimique du proton anomérique du sucrose octasulfate de potassium seul en solution ou dans la composition de l'exemple 4 sont identiques. Par conséquent, l'analyse RMN confirme que le sucrose octasulfate de potassium ne réagit pas avec l'acide hyaluronique pour former un complexe et que la composition de l'exemple 4, est un mélange physique d'une solution aqueuse de sucrose octasulfate de potassium et de l'hydrogel comprenant de l'acide hyaluronique réticulé.

La superposition des spectres RMN 1H de la figure 2 de la composition de l'exemple 4 avant et après stérilisation par autoclavage à la vapeur confirme la très grande identité des deux spectres. Les structures chimiques du sucrose octasulfate de potassium et de l'acide hyaluronique sont inchangées suite à la stérilisation par autoclavage à la vapeur, comme illustré dans la figure 2. Cela confirme les résultats concernant les propriétés rhéologiques de la composition de l'exemple 4 ci-dessus exposés.

EXEMPLE 12

Tests de dégradation enzymatique

Le test de dégradation a été mis au point sur la base du test décrit dans la publication «Comparison of the sensitivity of 11 crosslinked hyaluronic acid gels to bovine testis hyaluronidase », I. Sall, G. Ferard, Polymer Deg. and Stability, (2007), 92, 915-919 et la demande de brevet WO 2009/068215.

Les tests de dégradation enzymatique ont été réalisés sur une composition préparée selon le mode opératoire de l'exemple 2 comprenant du NaHA réticulé à une concentration de 20 mg/g et du sucrose octasulfate de potassium à une concentration de 3 mg/g dans du tampon NaCl (composition A) ainsi que sur la composition de l'exemple 4 (composition B), stérilisées par autoclavage à la vapeur et sur des compositions de référence également stérilisées par autoclavage à la vapeur. La composition de référence A et la composition de référence B sont formulées en remplaçant la solution aqueuse de sucrose octasulfate de potassium par une même quantité de solution aqueuse de tampon phosphate.

Les compositions ont été soumises à un test de dégradation enzymatique *in vitro* à 37°C. Ce test permet de simuler la rémanence ultérieure *in vivo* des compositions injectées.

Lesdites compositions sont dégradées par les hyaluronidases, en mélangeant les compositions à tester avec une solution de hyaluronidase.

La dégradation est suivie par rhéologie à 37°C (sur rhéomètre TA Instruments AR 2000 Ex), en mesurant la viscosité complexe. Les courbes de tendance des résultats de dégradation de ces deux compositions permettant ensuite d'évaluer le temps de demi-vie de ces différentes compositions (durée nécessaire pour avoir $n^* = n^*_0 / 2$, en minutes), avec $n^*_0$ = viscosité complexe à $t_0$ de la composition caractérisée.

Les temps de demi-vie obtenus sont donnés dans le tableau 4 ci-dessous :

Tableau 4

| **Composition** | **Dégradation enzymatique (Hyaluronidases)** |
|---|---|
| | **Temps ½ vie (min)** |
| Composition de référence A | 5,1 |
| Composition A | 6,3 |
| Composition de référence B | 6,1 |
| Composition B | 8,7 |

Tests de dégradation enzymatique sur une composition selon l'exemple 2 avec une concentration en KSOS de 3 mg/g (composition A) et sur la composition de l'exemple 4 (composition B), stérilisées par autoclavage à la vapeur et sur des compositions de référence à base d'acide hyaluronique réticulé stérilisée par autoclavage à la vapeur

Les compositions A et B, stérilisées par autoclavage à la vapeur présentent des temps de demi-vie supérieurs à ceux des compositions de référence, stérilisées par autoclavage à la vapeur dans les mêmes conditions; en effet les compositions A et B, stérilisées par autoclavage à la vapeur possèdent respectivement des rémanences supérieures de 24% et 43% vis-à-vis de la dégradation enzymatique par rapport aux compositions de référence A et B stérilisées par autoclavage à la vapeur.

Exemple 13

Tests de dégradation oxydante

Les tests de dégradation oxydante ont été réalisés sur la composition de l'exemple 4, stérilisée par autoclavage à la vapeur et sur une composition de référence également stérilisée par autoclavage à la vapeur dans les mêmes conditions. La composition de référence est formulée, en remplaçant la solution aqueuse de sucrose octasulfate de potassium par une même quantité de solution aqueuse de tampon phosphate.

Les compositions ont été soumises à un test de dégradation oxydante *in vitro* à 45°C. Ce test permet de simuler la rémanence ultérieure *in vivo* des compositions injectées.

Les compositions sont dégradées en présence d'eau oxygénée. La dégradation est suivie par rhéologie à 45°C (sur rhéomètre TA Instruments AR 2000 Ex), en mesurant la viscosité complexe. Les courbes de tendance des résultats de dégradation de ces compositions permettent ensuite d'évaluer le temps de demi-vie de ces différentes compositions

(durée nécessaire pour avoir n* = $n^*_0$ / 2, en minutes), avec $n^*_0$ = viscosité complexe à $t_0$ de la composition caractérisée.

Les temps de demi-vie obtenus sont donnés dans le tableau 5 ci-dessous :

Tableau 5

| **Oses** | **Dégradation oxydante ($H_2O_2$)** |
|---|---|
| | **Temps ½ vie (min)** |
| Composition de référence | 19,95 |
| Sucrose octasulfate de potassium | 25,85 |

Tests de dégradation oxydante sur une composition de l'exemple 4, stérilisée par autoclavage à la vapeur et sur une composition de référence à base d'acide hyaluronique réticulé, stérilisée par autoclavage à la vapeur

La composition de l'exemple 4, stérilisée par autoclavage à la vapeur présente un temps de demi-vie supérieur à celui de la composition référence stérilisée par autoclavage à la vapeur dans les mêmes conditions ; en effet, la composition de l'exemple 4, stérilisée par autoclavage à la vapeur possède une rémanence supérieure de 30% vis-à-vis de la dégradation oxydante par rapport à la composition de référence, stérilisée par autoclavage à la vapeur.

EXEMPLE 14

Exemples d'essais de formulations de KSOS (à différentes concentrations) avec un gel de NaHA réticulé à 20 mg/g (gel IPN like commercialisé sous la marque Stylage® M) qui est le gel de référence.

Les gels sont stérilisés puis caractérisés.

Le pourcentage de perte de la composante élastique G' par rapport à la composition de référence est mesuré selon l'exemple 8 et la différence entre la force d'injectabilité, à travers des aiguilles hypodermiques 30 Gauge 1/2, du gel de référence et des gels comprenant du sucrose octasulfate de potassium est mesurée.

Les résultats sont présentés dans le tableau 6 ci-dessous :

Tableau 6

| Stylage® M | % de perte de la composante élastique G' par rapport à la composition de référence | $\Delta_{\text{force d'Injectabilité}}$ (N) = Force d'injectabilité gel référence (N) - Force d'injectabilité gel avec KSOS (N) | [HA]/ [SOS] |
|---|---|---|---|
| Référence | | | |
| + KSOS à 3 mg/g | 0 | 0 | 6,66 |
| + KSOS à 1 mg/g | 0 | 0 | 20 |
| + KSOS à 0,5 mg/g | 0 | 0 | 40 |
| + KSOS à 0,2 mg/g | 0 | 0 | 100 |

Pourcentage de perte de la composante élastique G' après l'étape d'autoclavage à la vapeur de compositions comprenant de l'acide hyaluronique réticulé en présence de concentrations variables en sucrose octasulfate de potassium.

On n'observe aucune perte de la composante élastique G' après l'étape d'autoclavage des compositions comprenant l'acide hyaluronique réticulé et du sucrose octasulfate de potassium, quelle que soit la concentration en sucrose octasulfate de potassium dans la composition.

On n'observe également aucune augmentation de la force d'injectabilité par rapport à la formulation de référence quelle que soit la teneur en sucrose octasulfate de potassium.

EXEMPLE 15

Exemples d'essais de formulations de KSOS (à différentes concentrations) avec un gel non réticulé de l'exemple

1 aux concentrations et ratios décrits dans le tableau 7 ci-dessous.

Les gels sont stérilisés puis caractérisés par rhéologie selon l'exemple 8.

Tableau 7

| Concentration en NaHA (mg/g) | Concentration en KSOS (mg/g) | Ratio [HA]/[SOS] | % de perte de la viscosité par rapport à la composition de référence |
|---|---|---|---|
| 25 | 0 | - | - |
| 25 | 0,01 | 2500 | 0 |
| 25 | 0,05 | 500 | 0 |
| 20 | 0 | - | - |
| 20 | 10 | 2 | 0 |
| 20 | 20 | 1 | 0 |
| 10 | 0 | - | - |
| 10 | 10 | 1 | 0 |
| 10 | 20 | 0,5 | 0 |
| 4 | 0 | - | - |
| 4 | 40 | 0,1 | 0 |

Pourcentage de perte de la viscosité après l'étape d'autoclavage de compositions à différents ratios [HA]/[SOS].

On n'observe aucune perte de la viscosité après l'étape d'autoclavage des compositions, quel que soit le ratio [HA]/[SOS].

EXEMPLE 16

Exemple d'essai de formulation de KSOS avec un gel comprenant de l'acide hyaluronique réticulé à la concentration et au ratio décrit dans le tableau 8 ci-dessous.

Les gels sont préparés selon le mode opératoire décrit à l'exemple 2 puis caractérisés par rhéologie selon l'exemple 8.

Tableau 8

| Concentration en NaHA (mg/g) | Concentration en KSOS (mg/g) | Ratio [HA]/[SOS] | % de perte de la composante élastique G' par rapport à la composition de référence |
|---|---|---|---|
| 10,3 | 0 | - | - |
| 10,3 | 25 | 0,4 | 0 |

Pourcentage de perte de la composante élastique G' après l'étape d'autoclavage.

On n'observe aucune perte de la composante élastique G' après l'étape d'autoclavage des compositions, pour un ratio [HA]/[SOS] de 0,4.

## Revendications

1. Composition, stérilisée, comprenant, en milieu aqueux, au moins un acide hyaluronique et au moins un sel hydrosoluble de sucrose octasulfate, **caractérisée en ce que** ladite composition est un mélange physique et **en ce que** le ratio massique entre la teneur en acide hyaluronique [HA] et la teneur en sel hydrosoluble de sucrose octasulfate [SOS], [HA]/[SOS] est supérieur ou égal à 0,1.

2. Composition selon la revendication 1, **caractérisée en ce que** la masse moléculaire Mw de l'acide hyaluronique est comprise dans un intervalle de 0,01 MDa et 5 MDa et/ou la teneur en acide hyaluronique est comprise entre 2 mg/g et 50 mg/g de composition.

3. Composition selon l'une quelconque des revendications précédentes, **caractérisée en ce qu'**elle comprend au moins un acide hyaluronique non réticulé.

4. Composition selon l'une quelconque des revendications précédentes, **caractérisée en ce qu'**elle comprend au moins un acide hyaluronique réticulé.

5. Composition selon l'une quelconque des revendications précédentes, **caractérisée en ce que** le sel hydrosoluble de sucrose octasulfate est le sel de sodium de sucrose octasulfate ou le sel de potassium de sucrose octasulfate et la teneur en sel hydrosoluble de sucrose octasulfate est comprise entre 0,01 mg/g et 40 mg/g de composition.

6. Composition selon l'une quelconque des revendications précédentes, **caractérisée en ce qu'**elle comprend en outre, au moins un principe actif choisi parmi les antioxydants, les anesthésiques locaux, les vitamines, seuls ou en combinaison.

7. Composition selon la revendication 6, **caractérisée en ce que** les antioxydants sont choisis parmi les polyols.

8. Composition selon la revendication 6, **caractérisée en ce que** les anesthésiques locaux sont choisis dans le groupe constitué par la lidocaïne, la procaïne, la mépivacaïne, la ropivacaïne, la bupivacaïne, et leurs sels pharmaceutiquement acceptables.

9. Formulation cosmétique **caractérisée en ce qu'**elle comprend une composition selon les revendications 1 à 8, et au moins un excipient cosmétiquement acceptable.

10. Procédé de fabrication d'une composition selon les revendications 1 à 8, **caractérisé en ce qu'**il comprend au moins :

    • Une étape d'hydratation des fibres d'au moins un acide hyaluronique, pour obtenir un hydrogel,
    • Une étape de mélange d'une solution de sel hydrosoluble de sucrose octasulfate avec l'hydrogel obtenu à l'étape précédente,
    • Une étape d'homogénéisation, et
    • Une étape d'autoclavage à la vapeur.

11. Procédé de fabrication d'une composition selon la revendication 10, **caractérisé en ce qu'**il comprend en outre au moins une étape de réticulation.

12. Composition selon les revendications 1 à 8, destinée à être utilisée en viscosupplémentation.

13. Composition selon les revendications 1 à 8, destinée à être utilisée dans le comblement de rides.

14. Composition selon les revendications 1 à 8, destinée à être utilisée dans le traitement de la sécheresse oculaire.

15. Utilisation d'une composition selon les revendications 1 à 8, pour le comblement des rides.

16. Kit comprenant une composition selon les revendications 1 à 8, conditionnée en seringues ou en flacons uni-doses stérilisés.

**Patentansprüche**

1. Sterilisierte Zusammensetzung umfassend, in einem wässrigen Medium, wenigstens eine Hyaluronsäure und wenigstens ein wasserlösliches Salz von Saccharose-Octasulfat, **dadurch gekennzeichnet, dass** die Zusammensetzung eine physikalische Mischung ist und das Massenverhältnis aus dem Gehalt an Hyaluronsäure [HA] und dem Gehalt an wasserlöslichem Salz von Saccharose-Octasulfat [SOS], [HA]/[SOS] größer als oder gleich 0,1 ist.

2. Zusammensetzung nach Anspruch 1, **dadurch gekennzeichnet, dass** das Molekulargewicht MW der Hyaluronsäure in einem Bereich von 0,01 MDa und 5 MDa enthalten ist und/oder der Gehalt an Hyaluronsäure zwischen 2 mg/g Zusammensetzung und 50 mg/g Zusammensetzung enthalten ist.

3. Zusammensetzung nach einem der vorhergehenden Ansprüche, **dadurch gekennzeichnet, dass** sie wenigstens

eine nicht-quervernetzte Hyaluronsäure umfasst.

4. Zusammensetzung nach einem der vorhergehenden Ansprüche, **dadurch gekennzeichnet, dass** sie wenigstens eine quervernetzte Hyaluronsäure umfasst.

5. Zusammensetzung nach einem der vorhergehenden Ansprüche, **dadurch gekennzeichnet, dass** das wasserlösliche Salz von Saccharose-Octasulfat das Natriumsalz von Saccharose-Octasulfat oder das Kaliumsalz von Saccharose-Octasulfat ist und der Gehalt an wasserlöslichem Saccharose-Octasulfat zwischen 0,01 mg/g Zusammensetzung und 40 mg/g Zusammensetzung beträgt.

6. Zusammensetzung nach einem der vorhergehenden Ansprüche, **dadurch gekennzeichnet, dass** sie zusätzlich wenigstens einen aktiven Bestandteil umfasst, der ausgewählt ist aus Antioxidantien, Lokalanästhetika, Vitaminen, einzeln oder in Kombination.

7. Zusammensetzung nach Anspruch 6, **dadurch gekennzeichnet, dass** die Antioxidantien ausgewählt sind aus Polyolen.

8. Zusammensetzung nach Anspruch 6, **dadurch gekennzeichnet, dass** die Lokalanästhetika ausgewählt sind aus der Gruppe bestehend aus Lidocain, Procain, Mepivacain, Ropivacain, Bupivacain und ihren pharmazeutisch akzeptablen Salzen.

9. Kosmetische Formulierung, **dadurch gekennzeichnet, dass** sie eine Zusammensetzung nach einem der Ansprüche 1 bis 8 umfasst und wenigstens einen kosmetisch akzeptablen Hilfsstoff.

10. Verfahren zur Herstellung einer Zusammensetzung nach einem der Ansprüche 1 bis 8, **dadurch gekennzeichnet, dass** es wenigstens umfasst:

    - einen Schritt des Hydratisierens von Fasern aus wenigstens einer Hyaluronsäure, um ein Hydrogel zu erhalten,
    - einen Schritt des Mischens einer Lösung von wasserlöslichem Salz von Saccharose-Octasulfat mit dem im vorhergehenden Schritt erhaltenen Hydrogel,
    - einen Schritt des Homogenisierens, und
    - einen Schritt des Dampfautoklavierens.

11. Verfahren zur Herstellung einer Zusammensetzung nach Anspruch 10, **dadurch gekennzeichnet, dass** es weiterhin wenigstens einen Quervernetzungsschritt umfasst.

12. Zusammensetzung nach einem der Ansprüche 1 bis 8, vorgesehen für die Verwendung bei der Viskosupplementierung.

13. Zusammensetzung nach einem der Ansprüche 1 bis 8, vorgesehen zur Verwendung beim Auffüllen von Falten.

14. Zusammensetzung nach einem der Ansprüche 1 bis 8, vorgesehen zur Verwendung bei der Behandlung von Augentrockenheit.

15. Verwendung einer Zusammensetzung nach einem der Ansprüche 1 bis 8, für das Auffüllen von Falten.

16. Kit umfassend eine Zusammensetzung nach einem der Ansprüche 1 bis 8, verpackt in Spritzen oder in sterilisierten Einheitsdosenfläschchen.

## Claims

1. A sterilized composition including, in an aqueous medium, at least one hyaluronic acid and at least one sucrose octasulfate water-soluble salt, **characterized in that** said composition is a physical mixture and **in that** the weight ratio between the content of hyaluronic acid [HA] and the content of sucrose octasulfate water-soluble salt [SOS], [HA]/[SOS] is greater than or equal to 0.1.

2. The composition according to claim 1, **characterized in that** the molecular weight Mw of the hyaluronic acid is in

a range from 0.01 MDa to 5 MDa and/or the content of hyaluronic acid is from 2 mg/g to 50 mg/g of composition.

**3.** The composition according to either of the preceding claims, **characterized in that** it includes at least one non cross-linked hyaluronic acid.

**4.** The composition according to any one of the preceding claims, **characterized in that** it includes at least one cross-linked hyaluronic acid.

**5.** The composition according to any one of the preceding claims, **characterized in that** the sucrose octasulfate water-soluble salt is the sucrose octasulfate sodium salt or the sucrose octasulfate potassium salt, and the content of sucrose octasulfate water-soluble salt is from 0.01 mg/g to 40 mg/g of composition.

**6.** The composition according to any one of the preceding claims, **characterized in that** it further includes at least one active substance selected from the antioxidants, the local anesthetics, the vitamins, alone or in combination.

**7.** The composition according to claim 6, **characterized in that** the antioxidants are selected from the polyols.

**8.** The composition according to claim 6, **characterized in that** the local anesthetics are selected from the group consisting of lidocaine, procaine, mepivacaine, ropivacaine, bupivacaine and pharmaceutically acceptable salts thereof.

**9.** A cosmetic formulation **characterized in that** it includes a composition according to claims 1 to 8 and at least one cosmetically acceptable excipient.

**10.** A method for producing a composition according to claims 1 to 8, **characterized in that** it includes at least:

- a step of hydration of the fibers of at least one hyaluronic acid, in order to obtain a hydrogel,
- a step of mixing a solution of sucrose octasulfate water-soluble salt with the hydrogel obtained in the preceding step,
- a step of homogenization, and
- a steam autoclaving step.

**11.** The method for producing a composition according to claim 10, **characterized in that** it moreover includes at least one cross-linking step.

**12.** The composition according to claims 1 to 8, for use in viscosupplementation.

**13.** The composition according to claims 1 to 8, for use for filling wrinkles.

**14.** The composition according to claims 1 to 8, for use in the treatment of dry eye.

**15.** Use of a composition according to claims 1 to 8 for filling wrinkles.

**16.** A kit including a composition according to claims 1 to 8, packaged in syringes or in sterilized single-dose bottles.

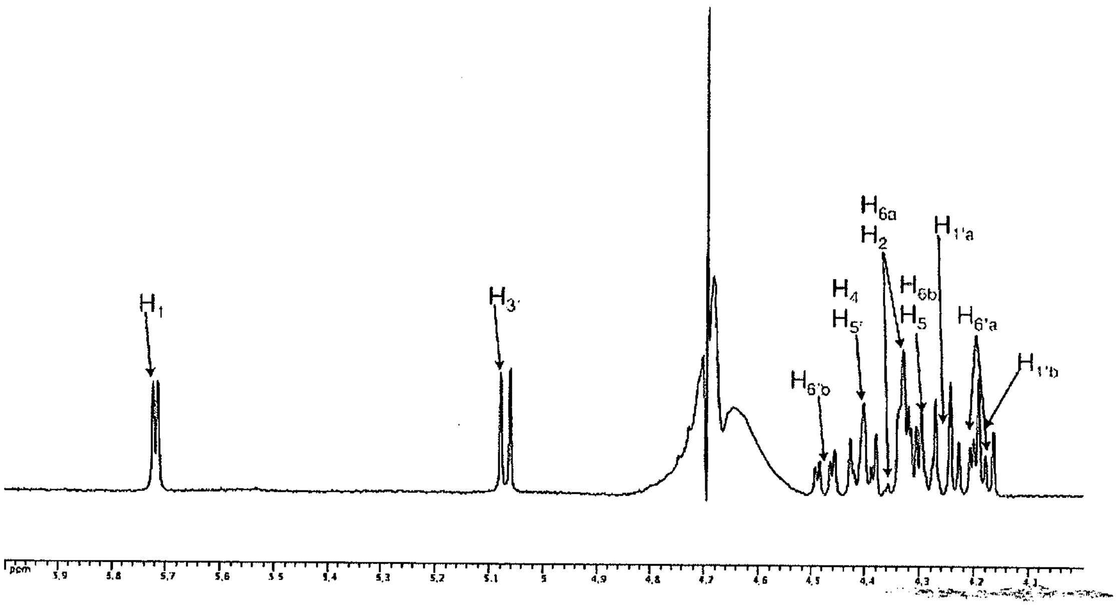


Figure 1

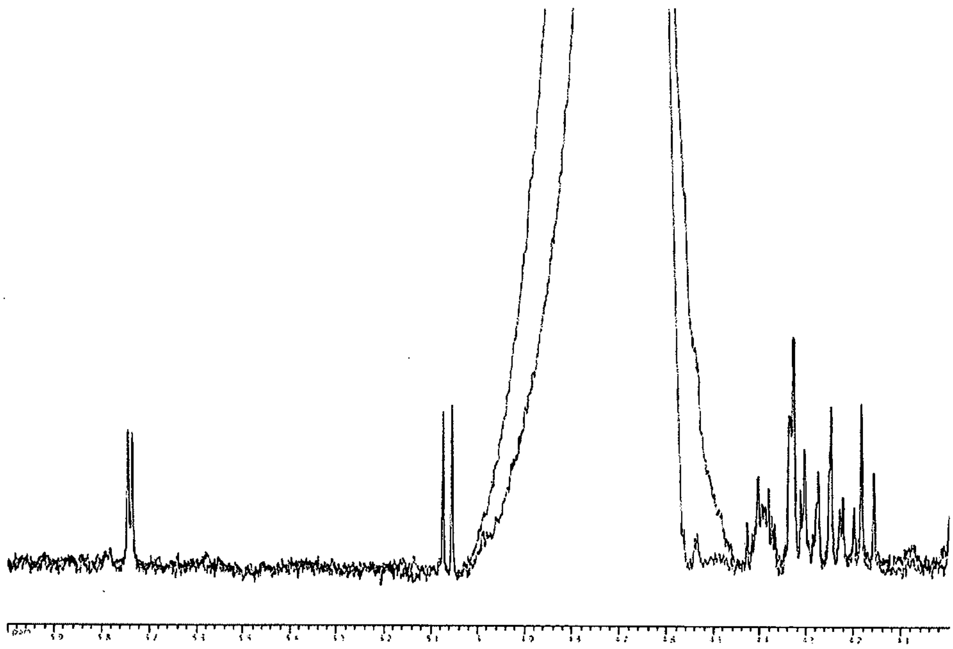

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03041724 A, Hermida
- WO 2004034980 A, Marcum
- US 2715091 A
- WO 03000191 A, Depuy
- EP 0230023 A, Marion
- WO 8905646 A
- EP 0640346 A, Bar-Shalom
- WO 9822114 A, Dumex-Alpharma
- WO 20000046253 A, Fermentech
- WO 2004092222 A, Cornéal
- FR 2865737
- WO 2005061611 A
- WO 071697 A
- WO 2013079889 A
- WO 2009071697 A
- WO 2009024670 A
- US 61791977 B
- FR 1352971
- WO 2009068215 A


### Littérature non-brevet citée dans la description


- **IOCONO et al.** *J. Ped. Surg.,* 1988, vol. 33 (4), 564-567
- **MOSELEY R et al.** *Biochim Biophys Acta,* 1995, vol. 1244 (2-3), 245-52
- **R. SERVATY et al.** *International Journal of Biological Macromolecules,* 2001, vol. 28, 121-127
- **T. TOIDA et al.** *Archives of Biochemistry and Biophysics,* 1999, vol. 370 (2), 176-182
- **K. ZIMMERMANN.** *J. Cancer Res. Clin. Oncol.,* 1983, vol. 105, 189-190
- **WALTON, BR.** *J. Pharmacol.,* 1954, vol. 9, 1-14
- **S. SALMEN et al.** *Planta Medica,* 2005, vol. 71 (8), 727-732
- **A. SUZUKI et al.** *Glycobiology,* 2001, vol. 11 (1), 57-64
- **J.I. FENTON et al.** *Osteoarthritis and Cartilage,* 2000, vol. 8 (6), 444-451
- **I. SALL ; G. FERARD.** Comparison of the sensitivity of 11 crosslinked hyaluronic acid gels to bovine testis hyaluronidase. *Polymer Deg. and Stability,* 2007, vol. 92, 915-919